# EUROPÄISCHE PATENTSCHRIFT

(11) **EP 0 586 987 B1**
(45) Veröffentlichungstag und Bekanntmachung des Hinweises auf die Patenterteilung: **06.05.1998**
(21) Anmeldenummer: 93113731.9
(22) Anmeldetag: 27.08.1993
(51) Int. Cl.: C07D 339/04, C07C 319/08, C07C 69/06

(54) **Verbessertes Verfahren zur Herstellung von R/S-gamma-Liponsäure oder R/S-alpha-Liponsäure**
Improved method for the preparation of R/S-gamma-lipoic acid or R/S-alpha-lipoic acid
Procédé amélioré pour la préparation de l'acide R/S-gamma-lipoique ou de l'acide R/S-alpha-lipoique

(30) Priorität: 08.09.1992 DE 4229914; 18.06.1993 DE 4320230
(43) Veröffentlichungstag der Anmeldung: 16.03.1994
(73) Patentinhaber: BASF Aktiengesellschaft, 67063 Ludwigshafen (DE)
(72) Erfinder: Paust, Joachim, Dr., D-67141 Neuhofen (DE); Eckes, Peter, Dr., D-67166 Otterstadt (DE); Siegel, Wolfgang, Dr., D-68167 Mannheim (DE); Balkenhohl, Friedhelm, Dr., D-67117 Limburgerhof (DE); Dobler, Walter, Dr., D-69126 Heidelberg (DE); Hüllmann, Michael, Dr., D-64646 Heppenheim (DE)

(56) Entgegenhaltungen:
- JOURNAL OF THE AMERICAN CHEMICAL SOCIETY, Bd. 79, Nr. 13 , 10. Juli 1957 Washington, DC, US, Seiten 3503 - 3505 A. SEGRE, ET AL.: 'A new synthesis of 6-thioctic acid (DL-alpha-lipoic acid)'
- CHEMICAL ABSTRACTS, vol. 95, no. 7, 17. August 1981, Columbus, Ohio, US; abstract no. 61574y, S.D. MEKHTIEV, ET AL.: 'Some features of the addition reaction of cyclic ketones to vinyl ethers' Seite 664 ; & AZERB. KHIM. ZH., 1980, (3), 54-57
- CHEMICAL ABSTRACTS, vol. 63, no. 12, 6. Dezember 1965, Columbus, Ohio, US; abstract no. 16355e, & JP-A-40 019 939 (KONGO CHEMICAL) 6. September 1965

## Beschreibung

Die Erfindung betrifft ein verbessertes Verfahren zur Herstellung von R/S-γ-Liponsäure (6,8-Dimercaptooctansäure) der allgemeinen Formel I oder R/S-α-Liponsaure (D,L-Thioctsäure) der Formel II 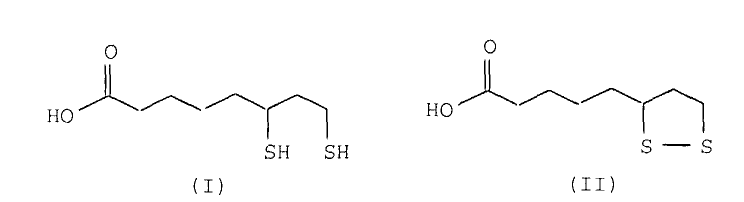 sowie neue Zwischenprodukte dieses Verfahrens.

R/S-α-Siponsäure ist ein Naturstoff, der in geringen Konzentrationen in praktisch allen tierischen und pflanzlichen Zellen vorkommt. Als Coenzym bei der oxidativen Decarboxylierung von α-Ketocarbonsäuren (z. B. Brenztraubensäure) ist α-Liponsäure von essentieller Bedeutung. Dieser körpereigene Wirkstoff wird als Razemat zur Behandlung von Lebererkrankungen und in zunehmendem Maße zur Behandlung von Neuropathien (besonders der diabetischen Polyneuropathie) eingesetzt. Nach neueren Ergebnissen (vgl. CA 116(21) : 2073606) kann α-Liponsäure möglicherweise Bedeutung bei der Bekämpfung durch HIV-1- und HTLV IIIB-Viren bedingter Krankheiten Bedeutung gewinnen. Auch der als Vorstufe von α-Liponsäure aufzufassenden γ-Liponsäure kommt neuerdings (vgl. DE 40 35 456) immer mehr Bedeutung zu. Es hat daher nicht an Versuchen gefehlt, ein technisch vorteilhaftes Verfahren zur Herstellung von γ-Liponsäure und/oder α-Liponsäure zu finden.

So wird z.B. in der Beschreibungseinleitung von DE 35 12 911 A1 eine Vielzahl von vielstufigen Verfahren zur Herstellung racemischer α-Liponsäure beschrieben, die größtenteils auf einer Umsetzung von Adipinsäuremonomethylesterchlorid mit Ethylen in Gegenwart von Aluminiumchlorid basieren.

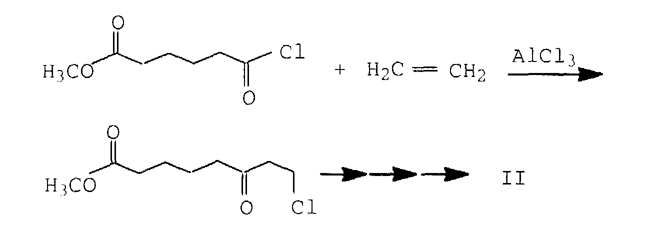

Das hierbei gebildete Chlorketon wird nach verschiedenen Verfahren über viele Stufen in Liponsäure überführt. Die Gesamtausbeute liegt hierbei bei maximal 30 %. Nachteilig an diesen Verfahren ist, daß sie von dem verhältnismäßig teuren Adipinsäuremonomethylesterchlorid ausgehen und, daß viele - teilweise energetisch oder bezüglich der verwendeten Reagentien - sehr aufwendige Verfahrensschritte durchlaufen werden müssen.

Auch das in der DE 35 12 911 A1 beanspruchte Verfahren zur Herstellung von γ-Liponsäure ausgehend von 2-(3-Alkylthiopropionyl)-cyclopentan-1-on nach dem folgenden Reaktionsschema läßt zu wünschen übrig.

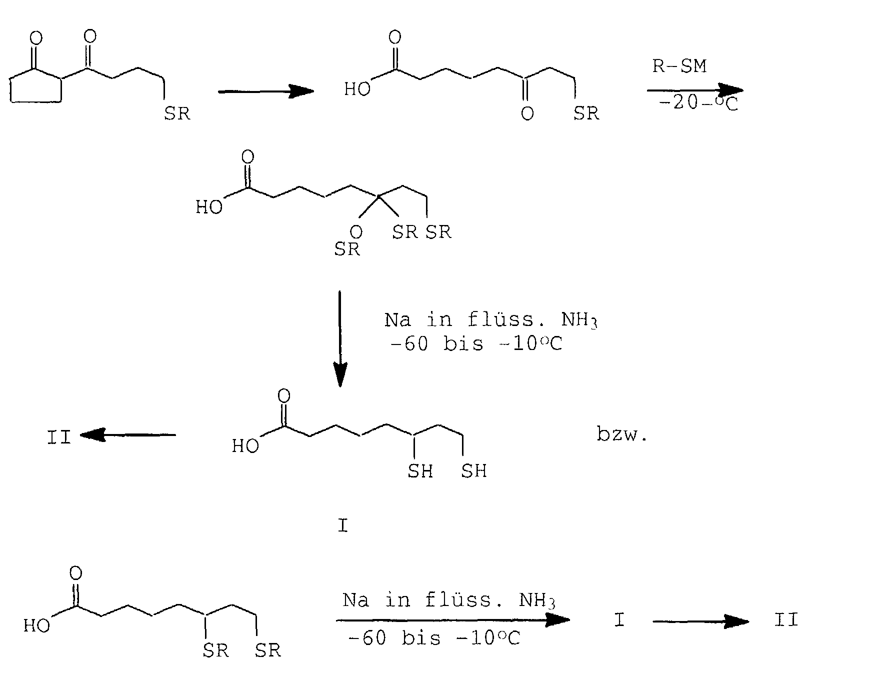

Nachteilig an diesem Verfahren ist, daß die Ausgangsverbindungen recht teuer sind, da sie in mehrstufigen Verfahren hergestellt werden müssen und auch die Umsetzung mit Natrium in flüssigem Ammoniak bei -60 bis -10°C hohe Kosten bedingt.

Weiterhin ist aus CA 63 (1965); Referat-Nr. 16 355 e die Herstellung von α-Liponsäure ausgehend von Cyclohexanon nach folgendem Reaktionsschema bekannt.

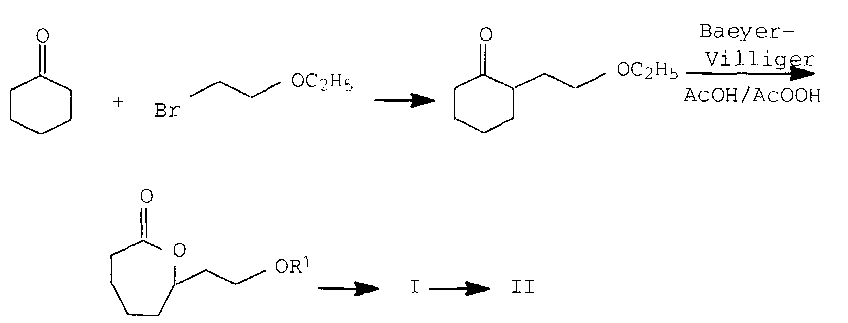

Nachteilig an diesem Verfahren ist, daß das als Ausgangsverbindung verwendete 2-Ethoxy-ethylbromid sehr teuer ist und daß insgesamt nur etwa 10 % Ausbeute an α-Liponsäure erzielt wird.

Ausbeuten von insgesamt nur etwa 19 %, bezogen auf Cyclohexanon, wurden gemäß dem Verfahren gemäß JACS 79 (1957) Seiten 3503 - 05 bzw. US 2,993,056 auf folgendem Wege erzielt:

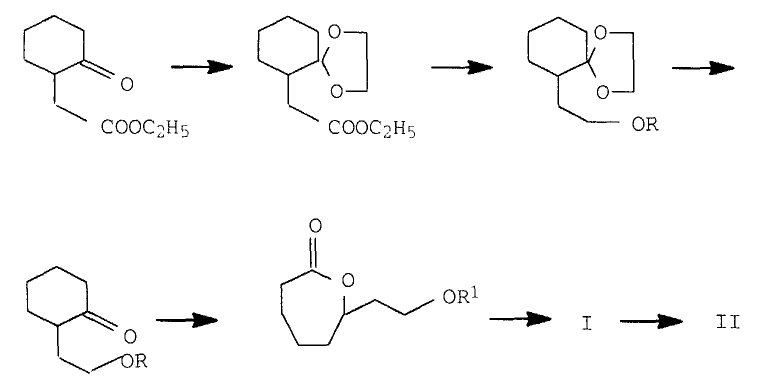

Es war daher die Aufgabe der Erfindung ein Verfahren zur Herstellung von R/S-α-Liponsäure zu finden, mit dem es gelingt, die R/S-α-Liponsäure ausgehend von einem billigen und gut zugänglichen Ausgangsprodukt in wenigen technisch leicht zu realisierenden Reaktionsstufen in guten Ausbeuten darzustellen. Es war weiterhin die Aufgabe der Erfindung auch für die Herstellung von R/S-γ-Liponsäure einen vorteilhaften Syntheseweg zu finden.

Gegenstand der Erfindung ist ein Verfahren zur Herstellung von R/S-γ-Liponsäure (6,8-Dimercapto-octansäure) der Formel I oder R/S-α-Liponsäure der Formel II 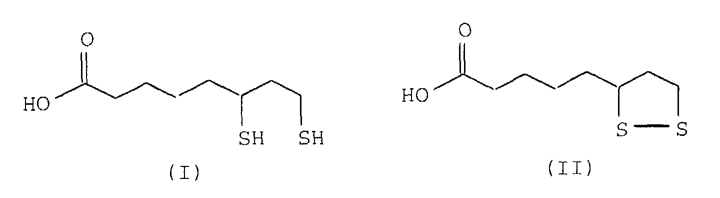 das dadurch gekennzeichnet ist, daß man
A. Cyclohexanon in Gegenwart eines geeigneten Radikalstarters mit einem Vinylalkylether der allgemeinen Formel III 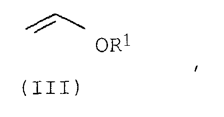 in der R¹ für C₁-C₃-alkyl steht,
   umsetzt,
B. das erhaltene 2-Alkoxyethyl-cyclohexanon der allgemeinen Formel IV 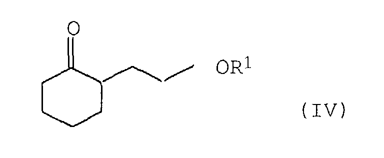 einer Baeyer-Villiger-Oxidation mit Perameisensäure in ein Gemisch bestehend im wesentlichen aus 8-Alkoxy-6-formyloxyoctansäure der allgemeinen Formel VII 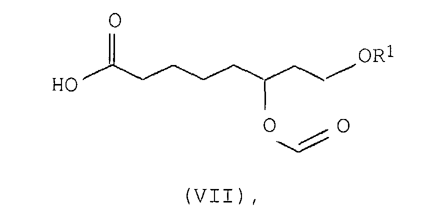 in der R¹ für C₁-C₃-alkyl steht, und geringen Mengen an 8-Alkoxy-6-hydroxy-octansäure der Formel VI und dem Lacton der Formel V 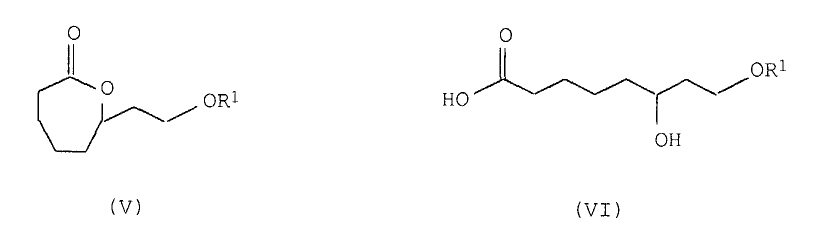 überführt und
C. das erhaltene Gemisch in an sich bekannter Weise in Gegenwart von wäßrigem Bromwasserstoff oder Jodwasserstoff mit Thioharnstoff zu R/S-γ-Liponsäure (6,8-Dimercapto-octansäure) der Formel I umsetzt und diese isoliert, oder
D. die erhaltene rohe R/S-γ-Liponsäure durch Luftoxidation in Gegenwart katalytischer Mengen von Eisen(III)verbindungen in die R/S-α-Liponsäure der Formel II überführt,
E. die erhaltene rohe R/S-α-Liponsäure einer kontinuierlichen Destillation in einem Filmverdampfer bei Drücken von 0,02 bis 0,2 mbar und Temperaturen von 60 bis 200°C unterwirft und
F. die so erhaltene destillativ vorgereinigte R/S-α-Liponsäure durch Kristallisation reinigt.

Besonders vorteilhaft gestaltet sich das erfindungsgemäße Verfahren wenn man im Reaktionsschritt A. das Cyclohexanon in Gegenwart von Di-tert.-butyl-peroxid mit dem Vinylalkylether der Formel III, insbesondere mit Vinylethylether, umsetzt.

Einen weiteren Vorteil erreicht man bei dem Gesamtverfahren wenn man im Reaktionsschritt C. das Gemisch aus 8-Alkoxy-6-formyloxyoctansäure der allgemeinen Formel VII, dem Lacton der Formel V und 8-Alkoxy-6-hydroxy-octansäure in Gegenwart von Bromwasserstoffsäure mit Thioharnstoff umsetzt und/oder im Reaktionsschritt D. die rohe R/S-γ-Liponsäure durch Luftoxidation in Gegenwart von Eisen(III)chlorid in die R/S-α-Liponsäure überführt, im Reaktionsschritt E. die R/S-α-Liponsäure bei Drücken von 0,05 bis 0,1 mbar und Temperaturen von 130 bis 160°C einer Destillation in einem Filmverdampfer unterwirft und im Verfahrensschritt F. die vorgereinigte R/S-α-Liponsäure durch Kristallisation aus Diisopropylether oder einem Gemisch aus Hexan und Essigsäureethylester reinigt.

Ganz besonders vorteilhaft gestaltet sich das Verfahren, wenn man im Reaktionsschritt B die Baeyer-Villiger-Oxidation mit Perameisensäure durchführt, die man in Gegenwart von 2-Alkoxyethylcyclohexanon aus Ameisensäure und Wasserstoffperoxid in situ erzeugt.

Das erfindungsgemäße Verfahren eröffnet einen besonders vorteilhaften Syntheseweg für R/S-γ-Liponsäure oder R/S-α-Liponsäure ausgehend von den leicht zugänglichen und daher sehr billigen Ausgangsverbindungen Cyclohexanon und Vinylalkylethern, insbesondere Vinylethylether.

So erhält man in der Stufe A durch radikalische Addition von Cyclohexanon an Vinylethylether das 2-Alkoxyethylcyclohexanon in Ausbeuten von etwa 70 % nach der Destillation.

Die radikalische Addition von Cyclohexanon an Vinylether wurde zwar schon im Azerb. Khim. Zh., (3) (1980) Seiten 54-57, in Ausbeuten von 50 bis 65 % beschrieben, jedoch in einer rein akademischen Studie und nicht im Zusammenhang mit der Herstellung von Liponsäure. Dagegen wurde in JA-AS 19939/65 die Herstellung von 2-Ethoxyethyl-cyclohexanon aus Cyclohexanon und 2-Ethoxy-ethylchlorid in einer Ausbeute von nur etwa 19 % beschrieben.

Die radikalische Addition von Cyclohexanon an Vinylacetat gemäß C.R. Acad. Sc. Paris, t. 289 (17.12.79) Seiten 445-47, ergab das 2-Acetoxyethyl-cyclohexanon nur in Ausbeuten von 20 %, so daß auch durch diese Arbeit nicht ein vorteilhafter Syntheseweg für α-Liponsäure ausgehend von Cyclohexanon möglich erschien.

Cyclohexanon wird in großem Umfang als Lösungsmittel oder als Ausgangsstoff für Polyamidsynthesen verwendet und steht daher technisch in großen Mengen billig zur Verfügung.

Die als Ausgangsstoffe für das erfindungsgemäße Verfahren weiterhin benötigten Vinylalkylether werden auf einfache Weise durch Addition von Alkoholen an Acetylen hergestellt.
- Zu A.: Als geeignete Radikalstarter für die radikalische Addition von Cyclohexanon an Vinylalkylether seien Dialkylperoxide genannt. Mit besonderem Vorteil arbeitet man mit Di-tert.- butylperoxid, da es besonders gut zugänglich und daher ein billiges Marktprodukt ist und bei Siedetemperatur des Cyclohexanons sofort Radikale bildet. Zur Durchführung der Umsetzung geht man im allgemeinen so vor, daß man Cyclohexanon bis zum Siedepunkt erhitzt und das siedende Cyclohexanon unter Rückflußkühlung langsam mit der Lösung des Radikalstarters in dem Vinylalkylether versetzt und das Reaktionsgemisch nach dem Ausreagierenlassen destillativ aufarbeitet. Das Cyclohexanon verwendet man dabei in Mengen von 5 bis 20, vorzugsweise 10 bis 15 Mol Cyclohexanon pro Mol Vinylalkylether. Den Radikalstarter verwendet man im allgemeinen in Mengen von 5 bis 50 Mol %, vorzugsweise 15-25 Mol % des Vinylalkylethers.
Die Umsetzung kann in einem inerten Lösungsmittel durchgeführt werden. Mit besonderem Vorteil arbeitet man jedoch ohne zusätzliches Lösungsmittel, d. h. in dem überschüssigen Cyclohexanon als Lösungsmittel.
Die Reaktionszeiten betragen im allgemeinen 2 bis 10, vorzugsweise 4 bis 6 Stunden. Die destillative Aufarbeitung des Reaktionsgemisches erfolgt unter vermindertem Druck.
- Zu B.: Die sogenannte Baeyer-Villiger-Oxidation von Alkoxyethylcyclohexanonen zu einem Lacton der allgemeinen Formel V bzw. einem Gemisch aus diesem Lacton und seinem Hydrolyseprodukt ist bereits aus C.A. 63 (1965) 16 355 g und JACS 79 (1957) Seiten 3503-05 bekannt. Bei der Durchführung dieser Oxidation geht man im allgemeinen so vor, daß man das 2-Alkoxyethylcyclohexanon bei Temperaturen von 0 bis 150°C (je nach Oxidationsmittel) mit einer organischen Persäure bzw. deren Salzen oder einer anorganischen Peroxoverbindung reagieren läßt.
Als ganz besonders gut geeignete Persäure für die Baeyer-Villiger-Oxidation hat sich Perameisensäure erwiesen, die man in Gegenwart des 2-Alkoxyethyl-cyclohexanon der Formel IV aus Ameisensäure und Wasserstoffperoxid in situ erzeugt.
Als Reaktionsgemisch dieser Umsetzung entsteht ein Gemisch aus einer 8-Alkoxy-6-formyloxy-octansäure der allgemeinen Formel VII 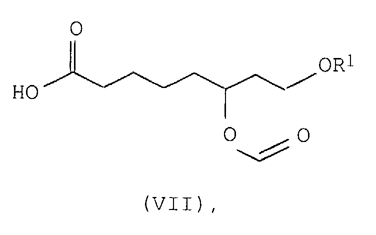 in der R¹ für C₁-C₃-alkyl steht, neben geringen Mengen an 8-Alkoxy-6-hydroxy-octansäure der Formel VI und deren Lacton der Formel V in Ausbeuten von 90 bis 95 % der Theorie.
8-Alkoxy-6-formyloxy-octansäuren der Formel VII sind bislang in der Literatur noch nicht beschrieben worden. Man kann sie aus dem oben beschriebenen Gemisch durch kontinuierliche Destillation unter stark vermindertem Druck, beispielsweise in einem Filmverdampfer, in einer Reinheit von mehr als 98 % erhalten. Sie können in reiner Form, aber mit besonderem Vorteil auch in Form des oben beschriebenen Gemisches durch Umsetzen mit Thioharnstoff in Gegenwart von Bromwasserstoffsäure oder Jodwasserstoffsäure (Reaktionsstufe C) in R,S-γ-Liponsäure der Formel I überführen, welche isoliert oder aber erfindungsgemäß in R,S-α-Liponsäure überführt werden kann, wobei die Ausbeute bei 90 bis 95 % der Theorie liegen.
Die Verwendung von Perameisensäure bringt zusätzlich zu den hervorragenden Ausbeuten noch große verfahrenstechnische und ökologische Vorteile mit sich. So werden bei der Baeyer-Villiger-Oxidation mit in situ erzeugter Perameisensäure, d.h. praktisch mit Ameisensäure und 30%ig wäßrigem Wasserstoffperoxid zwei Reaktionspartner eingesetzt, die preiswert und gefahrlos zu handhaben sind und zudem als Flüssigkeiten - im Gegensatz zu dem Feststoff Natrium-metaperborat - wesentlich leichter zu dosieren sind. Darüber hinaus ist es von Vorteil, daß man überschüssiges Oxidationsmittel einfach im Rahmen der Aufarbeitung thermisch zu CO₂ und H₂O zersetzen kann und daß man die 8-Alkoxy-6-formyloxy-octansäuren direkt weiterverarbeiten kann.
Baeyer-Villiger-Reaktionen mit Perameisensäure sind zwar im Prinzip bekannt (vgl. Comprehensive Organic Synthesis, Ed.B.M. Trost, Pergamon Press 1991, Vol. 7 p. 671; C. Grudzinski et al, J. Chem. Soc. Perkin I, 1182, 1978). In allen beschriebenen Beispielen entstehen jedoch aus cyclischen Ketonen die entsprechenden Lactone. Die von uns beobachtete nahezu quantitative Bildung der 8-Alkoxy-6-formyloxy-octansäuren ist neu und unerwartet.
Überraschend ist ebenfalls, daß die erfindungsgemäß hergestellten 8-Alkoxy-6-formyloxy-octansäuren nach Umsatz mit Bromwasserstoff/Thioharnstoff in Ausbeuten von 90 - 95 % d. Th. R,S-γ-Liponsäure liefern, während ausgehend vom Lacton der 8-Alkoxy-6-hydroxy-octansäure nur 70 - 80 % R,S-γ-Liponsäure erhalten werden.
Zur Durchführung der Baeyer-Villiger-Oxidation mit Perameisensäure löst man im allgemeinen das 2-Alkoxyethylcyclohexanon der Formel IV in Ameisensäure. Zu einer 1 bis 4 molaren, vorzugsweise einer 1,5 bis 2,5 molaren Lösung des 2-Alkoxyethyl-cyclohexanons in Ameisensäure fügt man dann Wasserstoffperoxid in Form einer vorzugsweise 30%igen wäßrigen Lösung in Mengen von 1,1 bis 1,8, vorzugsweise 1,4 bis 1,5 Mol pro Mol 2-Alkoxyethyl-cyclohexanon zu.
Der bevorzugte Temperaturbereich für die erfindungsgemäße Baeyer-Villiger-Reaktion liegt bei 10°C bis 70°C, vorzugsweise bei 40 bis 50°C. Insbesondere zu Beginn der Umsetzung muß der gewünschte Temperaturbereich durch externe Kühlung gewährleistet werden. Für die weitere Umsetzung zu R,S-γ-Liponsäure geeignete Präparate erhält man durch Abdestillieren des Ameisensäure/Wasser-Gemischs bei Normaldruck. Reine 8-Alkoxy-6-formyloxy-octansäure läßt sich aus dem Rohprodukt in einem Filmverdampfer isolieren.
- Zu C.: Die Überführung des Lactons der Formel V bzw. des Gemisches aus diesem Lacton und 8-Alkoxy-6-hydroxy-octansäure in die R/S-γ-Liponsäure der Formel I ist aus JACS 79 (1957), Seiten 3503-05, und C.A. 63 (1965) Referat Nr. 16355g, bekannt und erfolgt auf bekannte Weise. Erfindungsgemäß wird 8-Alkoxy-6-formyloxy-octansäure der allgemeinen Formel VII bzw. ein Gemisch dieser Säure mit dem Lacton der Formel V und 8-Alkoxy-6-hydroxy-octansäure mit ca. 8 Mol Thioharnstoff und ca. 7 Mol einer konz. Halogenwasserstoffsäure versetzt und für etwa 36 Stunden unter Rückfluß zum Sieden erhitzt. Anschließend wird das Reaktionsgemisch mit einer konz. Alkalihydroxidlösung versetzt und zur Vervollständigung der Hydrolyse noch etwa 12 Stunden unter Ausschluß von Luftsauerstoff und Licht unter Rückfluß zum Sieden erhitzt.
Anschließend wird mit HCl angesäuert und die R/S-γ-Liponsäure mit einem mit Wasser nicht oder nur schlecht mischbaren Lösungsmittel extrahiert. Die durch anschließendes Einengen erhaltene R/S-γ-Liponsäure kann entweder als solche isoliert oder aber oxidativ zu R/S-α-Liponsäure weiterverarbeitet werden.
- Zu D.: Zur Herstellung von R/S-α-Liponsäure wird das gemäß Stufe C erhaltene Gemisch in schwach alkalischem wäßrigem Milieu in Gegenwart von Eisen(III)-verbindungen mit Luftsauerstoff oxidiert. Auch diese Oxidation ist aus JACS 79 (1957), Seiten 3503-05, bekannt und erfolgt auf bekannte Weise. Mit Vorteil arbeitet man mit Eisen(III)-chlorid als Katalysator. Zur Durchführung der Oxidation wird für die Dauer der Reaktion Luftsauerstoff durch die wäßrig alkalische, die Eisen(III)-verbindung enthaltende Lösung durchgeleitet.
Die Reaktionstemperatur beträgt im allgemeinen 15 bis 40°C, die Reaktionszeit je nach Begasungsgeschwindigkeit 2 bis 10 Stunden. Als Eisen(III)-Verbindungen seien genannt: Eisen(III)-halogenide, insbesondere Eisen(III)-chlorid. Man verwendet sie im allgemeinen in katalytischen Mengen von 0,1 bis 0,5 Gew.-%, bezogen auf eingesetzte R/S-γ-Liponsäure.
Zur Aufarbeitung des Reaktionsgemisches wird nach der Oxidation angesäuert, mit einem mit Wasser nicht oder nur schlecht mischbaren Lösungsmittel extrahiert und der organische Extrakt getrocknet und eingeengt.
- Zu E.: Erfindungsgemäß wird die so erhaltene Roh-R/S-α-Liponsäure durch kontinuierliche Destillation in einem Filmverdampfer, d. h. einem Verdampfer mit kurzer Verweilzeit, unter stark vermindertem Druck destilliert. Man destilliert im allgemeinen bei Drucken von 0,02 bis 0,2, vorzugsweise 0,05 bis 0,1 mbar und entsprechend bei Temperaturen von 60 bis 200, vorzugsweise 130 bis 160°C ohne Zersetzung.
- Zu F.: Die so durch destillative Vorreinigung erhaltene R/S-α-Liponsäure kann dann durch Kristallisation aus Lösungsmitteln, wie Diisopropylether oder Lösungsmittelgemischen, wie Gemischen aus n-Hexan und Essigsäureethylester in sehr reiner Form erhalten werden.
Mit Hilfe des erfindungsgemäßen Verfahrens können R/S-γ-Liponsäure und R/S-α-Liponsäure auf relativ einfache Weise ausgehend von billigen Ausgangsstoffen in recht guten Ausbeuten erhalten werden. So erhält man R/S-α-Liponsäure in Ausbeuten von ca. 45 %, bezogen auf den eingesetzten Vinylalkylether.

### Beispiel 1

1) Herstellung von 2-(2-Ethoxyethyl)-cyclohexanon 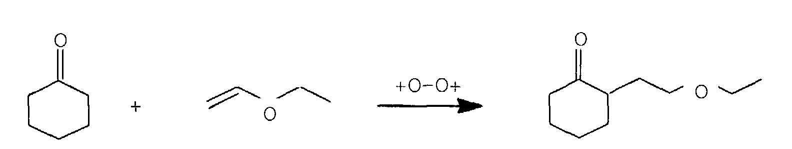 2940 g (30 mol) Cyclohexanon wurde unter Rühren bis zur Rückflußtemperatur erhitzt und bei dieser Temperatur im Verlauf von 5 Stunden (h) mit einer Lösung von 87,6 g (0,6 mol) Ditert.-butylperoxid in 216 g (3 mol) Ethylvinylether versetzt. Anschließend wurde das Reaktionsgemisch noch 1 h unter Rückfluß zum Sieden erhitzt. Nach dem Abkühlen des Reaktionsgemisches wurden die Niedrigsieder (tert.-Butanol, Reste des Vinylethers und Peroxid) sowie das überschüssige Cyclohexanon unter vermindertem Druck (25-40 mbar; Membranpumpe) abdestilliert. Der Rückstand (ca. 500 g) wurde unter vermindertem Druck (0,5 bis 2 mbar; Ölpumpe) destilliert. Insgesamt wurden 350 g eines 95 %igen 2-(2-Ethoxyethyl)-cyclohexanons vom Kp 60-65°C bei 0,5 mbar erhalten, entsprechend einer Ausbeute von 69 % der Theorie.
2) Herstellung des Lactons von 8-Alkoxy-6-hydroxy-octansäure 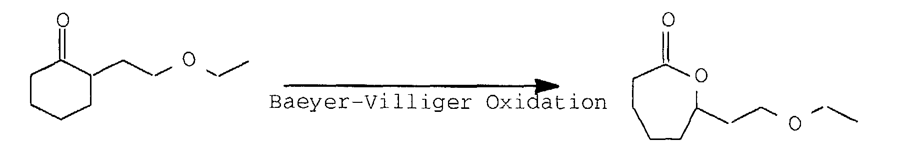
   a) mit 3-Chlor-perbenzoesäure als Oxidationsmittel (zum Vergleich)
      1506 g (4,8 mol) 3-Chlor-perbenzoesäure (55 %ig) wurden in 2000 ml CH₂Cl₂ suspendiert. 680 g (4 mol) eines gemäß 1) hergestellten 2-(2-Ethoxyethyl)-cyclohexanons wurden so langsam zu der Suspension getropft, daß die kräftig gerührte Mischung nicht zu heftig siedete (ca. 2 h; stark exotherme Reaktion). Anschließend wurde weitere 3 h bei 40°C gerührt. Die abgekühlte Reaktionsmischung wurde unter kräftigem Rühren langsam mit 3000 ml einer gesättigten Na₂CO₃-Lsg. versetzt. Nach Trennung der Phasen, erneutem Waschen der organischen Phase mit 2000 ml Na₂CO₃-Lsg., Trocknen über MgSO₄ und Einengen unter vermindertem Druck erhielt man 640 g des Lactons als farblose Flüssigkeit (95 % gemäß gaschromatographischer Analyse (GC), entsprechend einer Ausbeute von 86 % der Theorie.
   b) mit Natriumperborat als Oxidationsmittel (zum Vergleich)
      850 g (5 mol) 2-(2-Ethoxyethyl)-cyclohexanon wurden in 3 1 Essigsäuren gelöst. Die Lösung wurde auf Rückflußtemperatur erhitzt und langsam mit einer Suspension von 1150 g NaBO₂·H₂O₂·3H₂O (7,5 mol) in 2 l Essigsäure versetzt (exotherme Reaktion, Siedekühlung). Anschließend rührte man 1 h bei Siedetemperatur nach, kühlte auf 0°C ab und filtrierte ausgefälltes Natriumborat ab. Das Filtrat wurde bei 30 mbar/70°C eingeengt. Das zurückbleibende Rohprodukt (Gemisch aus 8-Ethoxy-6-hydroxy-octansäure und seinem Lacton) kann als solches direkt in die nächste Stufe eingesetzt werden.
3) Herstellung von R/S-γ-Liponsäure (Dihydroliponsäure; zum Vergleich) 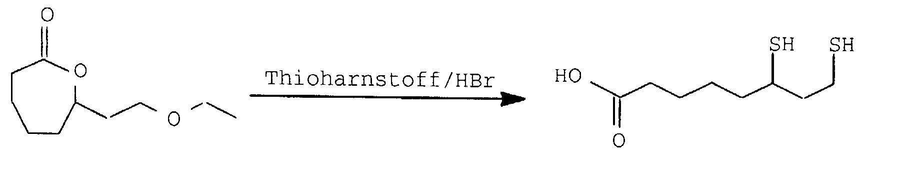 608 g (8 mol) Thioharnstoff wurden in 1205 g (7 mol) einer 47 %igen wäßrigen HBr gelöst und hierzu 186 g (1 mol) eines gemäß 2a) hergestellten Lactons von 8-Ethoxy-6-hydroxyoctansäure wurden zugesetzt. Die Mischung wurde anschließend 36 h unter Schutzgas und Rückflußkühlung zum Sieden erhitzt (105°C). Nach Abkühlung auf 50°C wurde die klare, schwach gelbe Lösung innerhalb von 1 h langsam mit 3200 g (20 mol) einer 35 %igen wäßrigen KOH versetzt und die erhaltene Reaktionslösung weitere 10 h im Dunkeln unter Rückfluß zum Sieden erhitzt. Während dieser Zeit sowie auch beim nachfolgenden Ansäuern leitete man einen Stickstoffstrom durch das Reaktionsgefäß, der anschließend durch zwei Waschtürme (A: gefüllt mit 4000 g einer 20 %igen HCl; B: gefüllt mit einer Mischung aus 3000 g einer 10 %igen NaOH und 3000 g einer 10 %igen NaOCl-Lösung) geleitet wurde. Nach Abkühlung wurde das Reaktionsgemisch mit ca. 1100 g konz. HCl angesäuert (pH=1) und die rohe R/S-γ-Liponsäure durch dreimaliges Extrahieren mit je 1000 ml Methyl-tert.-butyl-ether abgetrennt. Nach Trocknen über MgSO₄ und Einengen erhielt man 210 g rohe R/S-γ-Liponsäure als schwach gelbes Öl mit einem Gehalt von 70-80 % α + γ-Liponsäure nach GC.
4) Herstellung von R/S-α-Liponsäure 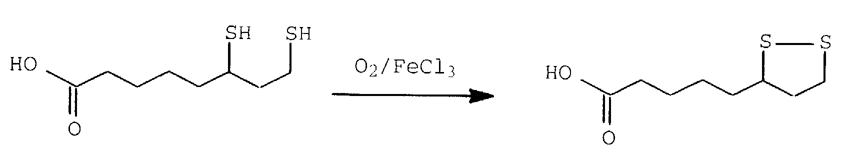 200 g der gemäß 3) erhaltenen rohen R/S-γ-Liponsäure wurden in ca. 525 ml 2N NaOH gelöst (pH = 8-9). Man verdünnte mit 3600 ml H₂O und versetzte die erhaltene Lösung mit 4 ml einer 10 %igen Lösung von FeCl₃·6H₂O. Anschließend leitete man so lange Luft durch die tiefrote Lösung bis die Farbe nach blaßgelb umschlug (ca. 3 h). Nach Zugabe von 1 l Methyl-tert.-butyl-ether wurde der pH-Wert mit konz. HCl auf ca. 1,5 eingestellt. Nach Phasentrennung wurde die Wasserphase noch zweimal mit je 500 ml Methyl-tert.-butyl-ether extrahiert. Die vereinigten organischen Phasen wurden getrocknet und eingeengt. Die rohe R/S-α-Liponsäure wurde an einem Filmverdampfer destilliert. Man erhielt 160 g vom Kp = 150°C bei 0,1 mbar als gelbes Kristallisat. Zweimaliges Umkristallisieren aus Diisopropylether lieferte 108 g reine R/S-α-Liponsäure in Form schwachgefärbte Kristalle vom Fp: 61°C. GC-Gehalt > 99 %, entsprechend einer Ausbeute von 55,3 %, bezogen auf eingesetztes Lacton der 8-Ethoxy-6-hydroxy-octansäure.

### Beispiel 2

a) Baeyer-Villiger-Oxidation mit in situ hergestellter Perameisensäure 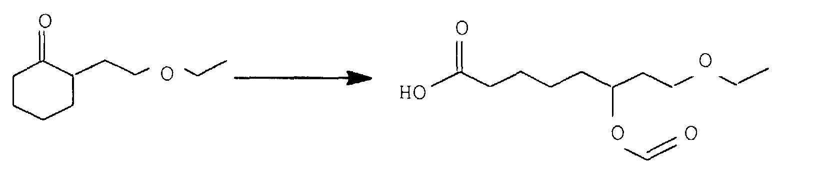
   Zu einer Lösung von 340 g (2 mol) 2-Ethoxyethyl-cyclohexanon in 1 l Ameisensäure wurden in etwa 1 Stunde (h) 340 g (3 mol) eines 30 %ig. wäßrigen Wasserstoffperoxids zugefügt und das Reaktionsgemisch noch 1 h nachreagieren lassen. Die Innentemperatur wurde während dieser Zeit bei 45 ± 5°C gehalten.
   Zur Aufarbeitung erhitzte man das Reaktionsgemisch innerhalb von etwa 1 h auf 100°C, wobei sich überschüssiges Oxidationsmittel unter CO₂-Entwicklung zersetzte. Danach destillierte man Ameisensäure und Wasser bei leicht vermindertem Druck und einer Badtemperatur von ca. 100°C ab.
   Der Rückstand bestand nach HPLC-Analyse zu 90 - 95 % aus 8-Ethoxy-6-formyloxy-octansäure neben geringen Mengen an 8-Ethoxy-6-hydroxy-octansäure, dem entsprechenden Lacton sowie einem Dimeren vor. 8-Ethoxy-6-formyloxy-octansäure mit einer Reinheit von > 98 % erhielt man durch kontinuierliche Destillation des Rohprodukts in einem Filmverdampfer bei 130°C, 0,05 mbar.
b) Herstellung von R,S-γ-Liponsäure 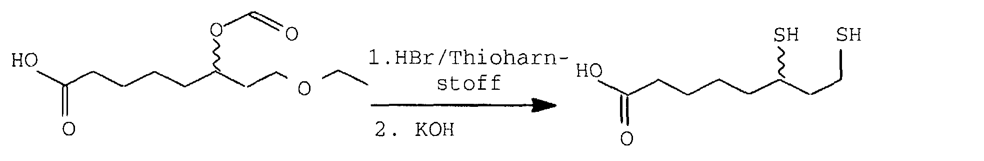 608 g (8 mol) Thioharnstoff wurden in 1205 g (7 mol) einer 47 %ig. wäßrigen Bromwasserstoffsäure gelöst. Dazu gab man 242 g (ca. 1 mol) der gemäß Beispiel 2a hergestellten rohen 8-Ethoxy-6-formyloxy-octansäure und erhitzte die Mischung 36 h unter Rückflußkühlung zum Sieden.

Die klare schwachgelbe Lösung der gebildeten Thiuroniumsalze ließ man unter Inertgas und Lichtausschluß innerhalb 1 h in 3200 g (20 mol) einer 35%ig. wäßrigen Kalilauge einlaufen und erhitzte das Reaktionsgemisch 8 h unter Rückflußkühlung zum Sieden. Durch das Reaktionsgefäß leitete man während dieser Zeit sowie beim nachfolgenden Ansäuern einen Inertgasstrom der anschließend zwei Waschtürmen (W) zugeführt wurde. WI war gefüllt mit 4000 g einer 20%ig. Salzsäure, WII mit einer Mischung aus 3000 g einer 10%ig. Natronlauge und 3000 g einer 10%ig. Natriumhypochloritlösung.

Nach Abkühlung auf 25°C wurde das Reaktionsgemisch mit ca. 1100 g konz. Salzsäure bis zu einem pH = 1-2 angesäuert und die R,S-γ-Liponsäure durch dreimaliges Extrahieren mit je 1000 ml Methyl-tert.-butylether abgetrennt. Nach Einengen erhielt man 218 g rohe R,S-γ-Liponsäure als schwach gelbes Öl, das sich für die weitere Umsetzung zu R,S-α-Liponsäure eignet.

Das Produkt wurde durch kontinuierliche Destillation in einem Filmverdampfer (150°C, 0,1 mbar) von Lösungsmittelresten und nicht-flüchtigen Bestandteilen befreit. Nach GC- und HPLC-Analyse besteht das Destillat (195 g) aus einem 9/1 Gemisch von R,S-γ-Liponsäure und R,S-α-Liponsäure; dies entspricht einer Gesamtausbeute an Wertprodukt von 93,7 % der Theorie.

## Patentansprüche

1. Verfahren zur Herstellung von R/S-γ-Liponsäure (6,8-Dimercaptooctansäure) der Formel I oder R/S-α-Liponsäure der Formel II 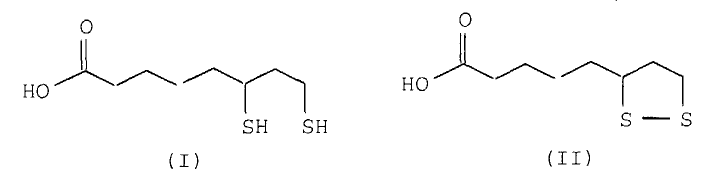 dadurch gekennzeichnet, daß man
A. Cyclohexanon in Gegenwart eines geeigneten Radikalstarters mit einem Vinylalkylether der allgemeinen Formel III 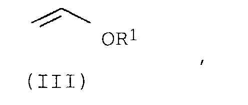 in der R¹ für C₁-C₃-alkyl steht,
umsetzt,
B. das erhaltene 2-Alkoxyethyl-cyclohexanon der allgemeinen Formel IV 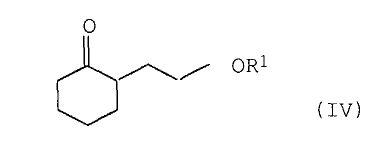 durch Baeyer-Villiger-Oxidation mit Perameisensäure in ein Gemisch aus 8-Alkoxy-6-formyloxy-octansäure der allgemeinen Formel VII 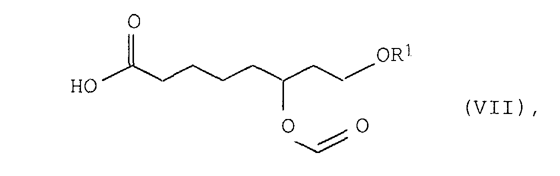 in der R¹ für C₁-C₃-alkyl steht, und geringen Mengen an 8-Alkoxy-6-hydroxy-octansäure der Formel VI und deren Lacton der Formel V 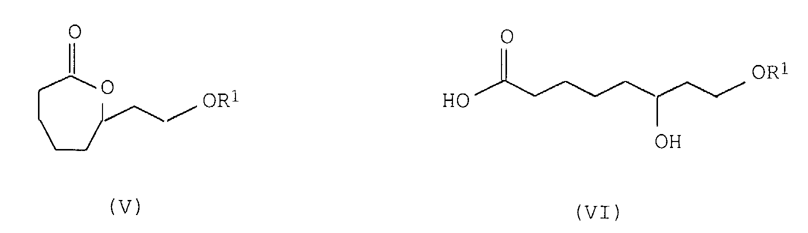 überführt,
C. das erhaltene Gemisch in an sich bekannter Weise in Gegenwart von Bromwasserstoffsäure oder Jodwasserstoffsäure mit Thioharnstoff zu R/S-γ-Liponsäure (6,8-Dimercaptooctansäure) der Formel I umsetzt und diese isoliert, oder
D. die erhaltene rohe R/S-γ-Liponsäure durch Luftoxidation in Gegenwart katalytischer Mengen von Eisen(III)verbindungen in die R/S-α-Liponsäure der Formel II überführt,
E. die erhaltene rohe R/S-α-Liponsäure einer kontinuierlichen Destillation in einem Filmverdampfer bei Drücken von 0,02 bis 0,2 mbar und Temperaturen von 60-200°C unterwirft und
F. die so erhaltene destillativ vorgereinigte R/S-α-Liponsäure durch Kristallisation isoliert.

2. Verfahren gemäß Anspruch 1, dadurch gekennzeichnet, daß man im Reaktionsschritt A. das Cyclohexanon in Gegenwart von Ditert.-butyl-peroxid mit dem Vinylalkylether der Formel III umsetzt.

3. Verfahren gemäß Anspruch 1, dadurch gekennzeichnet, daß man im Reaktionsschritt B,das 2-Alkoxyethyl-cyclohexanon in Form einer 1 bis 4 molaren Lösung in Ameisensäure mit Wasserstoffperoxid als in situ hergestellter Perameisensäure umsetzt.

4. Verfahren gemäß Anspruch 1, dadurch gekennzeichnet, daß man im Reaktionsschritt D. die rohe R/S-γ-Liponsäure durch Luftoxidation in Gegenwart von Eisen(III)-chlorid in die R/S-α-Liponsäure überführt.

5. Verfahren gemäß Anspruch 1, dadurch gekennzeichnet, daß man im Reaktionsschritt E.die erhaltene rohe R/S-α-Liponsäure bei Drücken von 0,05 bis 0,1 mbar und Temperaturen von 130 bis 160°C destilliert.

6. Verfahren gemäß Anspruch 1, dadurch gekennzeichnet, daß man im Verfahrensschritt F. die vorgereinigte R/S-α-Liponsäure durch Kristallisation aus Diisopropylether oder einem Gemisch aus Hexan und Essigsäureethylester reinigt.

7. Verfahren zur Herstellung von R/S-γ-Liponsäure oder R/S-α-Liponsäure gemäß Anspruch 1, dadurch gekennzeichnet, daß man im
Reaktionsschritt A das Cyclohexanon in Gegenwart von Di-tert.-butylperoxid als Radikalstarter mit dem Vinylalkylether der allgemeinen Formel III umsetzt,
im
Reaktionsschritt B die Baeyer-Villiger-Oxidation des 2-Alkoxyethyl-cyclohexanons der allgemeinen Formel IV mit in situ hergestellter Perameisensäure durchführt,
im
Reaktionsschritt C das Gemisch aus 8-Alkoxy-6-formyloxy-octansäure der Formel VII und geringen Mengen an dem Lacton von 6-Hydroxy-8-alkoxyoctansäure der Formel V und 6-Hydroxy-8-alkoxy-octansäure der Formel VI in Gegenwart von konzentrierter Bromwasserstoffsäure mit Thioharnstoff zu R/S-γ-Liponsäure umsetzt und diese isoliert,
oder im
Reaktionsschritt D die erhaltene rohe R/S-γ-Liponsäure durch Luftoxidation in Gegenwart katalytischer Mengen von Eisen(III)-chlorid in die R/S-α-Liponsäure überführt
und im
Reaktionsschritt E die erhaltene R/S-α-Liponsäure durch kontinuierliche Destillation in einem Filmverdampfer bei einem Druck von 0,05 bis 0,1 mbar und Temperaturen von 130 bis 160°C destilliert
und im
Reaktionsschritt F die destillativ vorgereinigte R/S-α-Liponsäure durch Kristallisation aus Diisopropylether oder einem Gemisch aus Hexan und Essigsäureethylester reinigt.

8. 8-Alkoxy-6-formyloxy-octansäure der allgemeinen Formel VII 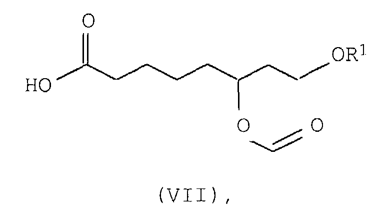 in der R¹ für C₁-C₃-alkyl steht.

9. Verfahren zur Herstellung der 8-Alkoxy-6-formyloxy-octansäure der allgemeinen Formel VII 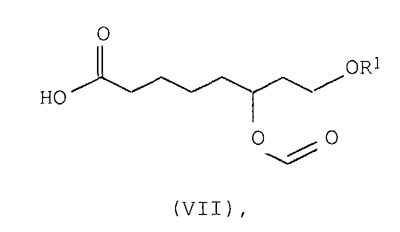 in der R¹ für C₁-C₃-alkyl bedeutet,
dadruch gekennzeichnet, daß man ein 2-Alkoxyethyl-cyclohexanon der allgemeinen Formel IV in Ameisensäure mit Wasserstoffperoxid umsetzt.

## Claims

1. A process for preparing R/S-γ-lipoic acid (6,8-dimercaptooctanoic acid) of the formula I or R/S-α-lipoic acid of the formula II 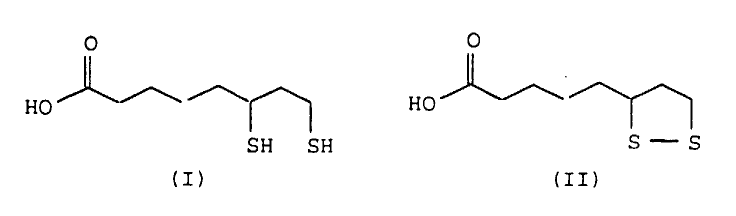 which comprises
A. reacting cyclohexanone in the presence of a suitable free radical initiator with a vinyl alkyl ether of the formula III 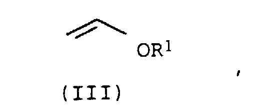 where R¹ is C₁-C₃-alkyl,
B. converting the resulting 2-alkoxyethylcyclohexanone of the formula IV 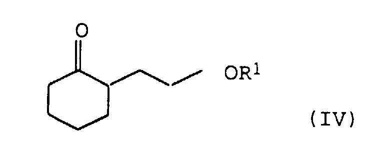 by Baeyer-Villiger oxidation with performic acid into a mixture of 8-alkoxy-6-formyloxy-octanoic acid of the formula VII 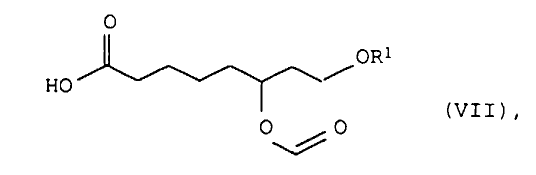 where R¹ is C₁-C₃-alkyl, and small amounts of 8-alkoxy-6-hydroxyoctanoic acid of the formula VI and its lactone of the formula V 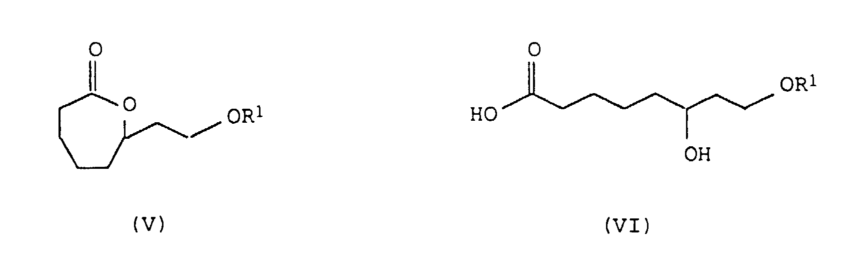
C. reacting the resulting mixture in a conventional manner in the presence of hydrobromic acid or hydriodic acid with thiourea to give R/S-γ-lipoic acid (6,8-dimercaptooctanoic acid) of the formula I, and isolating the latter, or
D. converting the resulting crude R/S-γ-lipoic acid by aerial oxidation in the presence of catalytic amounts of iron(III) compounds into the R/S-α-lipoic acid of the formula II,
E. subjecting the resulting crude R/S-α-lipoic acid to a continuous distillation in a thin film evaporator under 0.02 - 0.2 mbar and at 60-200°C, and
F. crystallizing the resulting R/S-α-lipoic acid distillate.

2. A process as claimed in claim 1, wherein the cyclohexanone is reacted in step A. with the vinyl alkyl ether of the formula III in the presence of di-tert-butyl peroxide.

3. A process as claimed in claim 1, wherein the 2-alkoxyethylcyclohexanone is reacted in step B. in the form of a 1-4 molar solution in formic acid with hydrogen peroxide as performic acid prepared in situ.

4. A process as claimed in claim 1, wherein the crude R/S-γ-lipoic acid is converted in step D. by aerial oxidation in the presence of iron(III) chloride into R/S-α-lipoic acid.

5. A process as claimed in claim 1, wherein the crude R/S-α-lipoic acid is distilled in step E. under from 0.05 to 0.1 mbar and at from 130 to 160°C.

6. A process as claimed in claim 1, wherein the R/S-α-lipoic acid is purified in step F. by crystallization from diisopropyl ether or a mixture of hexane and ethyl acetate.

7. A process for preparing R/S-γ-lipoic acid or R/S-α-lipoic acid as claimed in claim 1, wherein
in step. A. the cyclohexanone is reacted in the presence of di-tert-butyl peroxide as free radical initiator with the vinyl alkyl ether of the formula III,
in step B. the Baeyer-Villiger oxidation of the 2-alkoxyethylcyclohexanone of the formula IV is carried out with performic acid prepared in situ,
in step C. the mixture of 8-alkoxy-6-formyloxyoctanoic acid of the formula VII and small amounts of the lactone of 8-alkoxy-6-hydroxyoctanoic acid of the formula V and 8-alkoxy-6-hydroxyoctanoic acid of the formula VI is reacted in the presence of concentrated hydrobromic acid with thiourea to give R/S-γ-lipoic acid, and the latter is isolated, or
in step D. the resulting crude R/S-γ-lipoic acid is converted by aerial oxidation in the presence of catalytic amounts of iron(III) chloride into R/S-α-lipoic acid, and
in step E. the resulting R/S-α-lipoic acid is distilled continuously in a thin film evaporator under from 0.05 to 0.1 mbar and at from 130 to 160°C, and
in step F. the R/S-α-lipoic acid distillate is crystallized from diisopropyl ether or a mixture of hexane and ethyl acetate.

8. 8-Alkoxy-6-formyloxyoctanoic acid of the formula VII 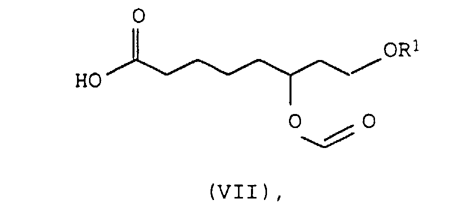 where R¹ is C₁-C₃-alkyl.

9. A process for preparing 8-alkoxy-6-formyloxyoctanoic acid of the formula VII 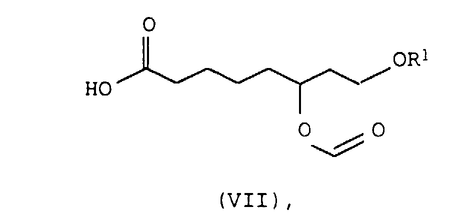 where R¹ is C₁-C₃-alkyl, which comprises reacting a 2-alkoxyethylcyclohexanone of the formula IV in formic acid with hydrogen peroxide.

## Revendications

1. Procédé pour préparer l'acide R/S-gamma-liponique (acide 6,8-dimercaptooctanoïque) de formule I ou l'acide R/S-alpha-liponique de formule II 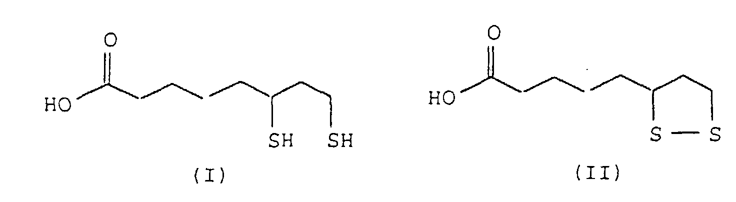 caractérisé par le fait que
A. on fait réagir la cyclohexanone en présence d'un inducteur radicalaire approprié avec un éther vinylalkylique de formule générale III 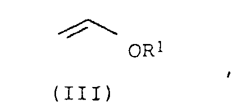 dans laquelle R¹ représente un groupe alkyle en C1-C3,
B. on convertit la 2-alcoxyéthyl-cyclohexanone ainsi obtenue, de formule générale IV 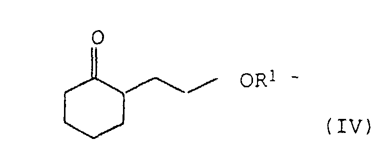 par une oxydation de Baeyer-Villiger à l'aide de l'acide performique, en un mélange de l'acide 8-alcoxy-6-formyloxy-octanoïque de formule générale VII 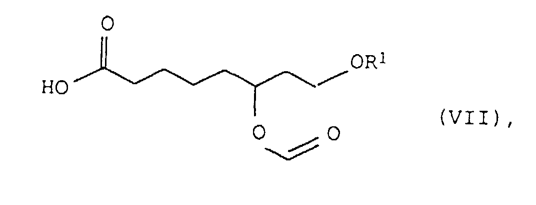 dans laquelle R¹ représente un groupe alkyle en C1-C3, et de petites quantités de l'acide 8-alcoxy-6-hydroxy-octanoïque de formule VI et de sa lactone de formule V 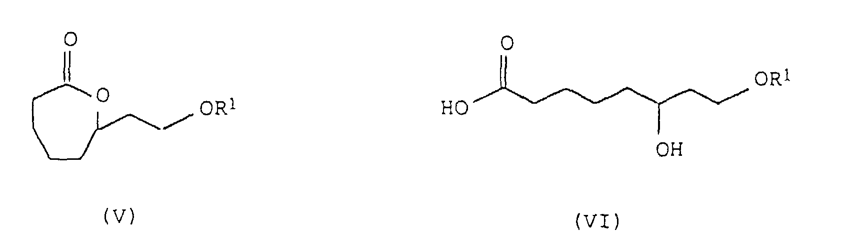
C. on convertit ce mélange, de manière connue en soi, en présence d'acide bromhydrique ou d'acide iodhydrique et à l'aide de la thiourée, en l'acide R/S-gamma-liponique (acide 6,8-dimercapto-octanoïque) de formule I qu'on isole, ou bien
D. on convertit l'acide R/S-gamma-liponique brut ainsi obtenu, par oxydation à l'air en présence de quantités catalytiques de composé du fer-III, en l'acide R/S-alpha-liponique de formule II,
E. on soumet l'acide R/S-alpha-liponique brut ainsi obtenu à une distillation continue dans un évaporateur à pellicule, sous des pressions de 0,02 à 0,2 mbar et à des températures de 60 à 200°C et
F. on isole par cristallisation l'acide R/S-alpha-liponique purifié au préalable par distillation.

2. Procédé selon la revendication 1, caractérisé par le fait que, au stade de réaction A., on fait réagir la cyclohexanone avec l'éther vinylalkylique de formule III en présence de peroxyde de di-tert-butyle.

3. Procédé selon la revendication 1, caractérisé par le fait que, au stade de réaction B., on fait réagir la 2-alcoxyéthyl-cyclohexanone sous forme d'une solution 1M à 4M dans l'acide formique, avec le peroxyde d'hydrogène, formant ainsi in situ l'acide performique.

4. Procédé selon la revendication 1, caractérisé par le fait que, au stade de réaction D., on convertit l'acide R/S-gamma-liponique brut en l'acide R/S-alpha-liponique par oxydation par l'air en présence de chlorure de fer-III.

5. Procédé selon la revendication 1, caractérisé par le fait que, au stade de réaction E., on soumet l'acide R/S-alpha-liponique brut à distillation sous des pressions de 0,05 à 0,1 mbar et à des températures de 130 à 160°C.

6. Procédé selon la revendication 1, caractérisé par le fait que, au stade opératoire F., on purifie l'acide R/S-alpha-liponique qui a déjà subi la purification préalable à purification finale par cristallisation dans l'éther diisopropylique ou dans un mélange d'hexane et d'acétate d'éthyle.

7. Procédé pour préparer l'acide R/S-gamma-liponique ou l'acide R/S-alpha-liponique selon la revendication 1, caractérisé par le fait que
au stade de réaction A
on fait réagir la cyclohexanone en présence de peroxyde de di-tert-butyle consistant l'inducteur radicalaire avec l'éther vinylalkylique de formule générale III,
au stade de réaction B
on procède à l'oxydation de Baeyer-Villiger de la 2-alcoxyéthylcyclohexanone de formule générale IV à l'aide d'acide performique préparé in situ,
au stade de réaction C
on fait réagir le mélange de l'acide 8-alcoxy-6-formyloxy-octanoïque de formule VII et de petites quantités de la lactone de l'acide 6-hydroxy-8-alcoxy-octanoïque de formule V et de l'acide 6-hydroxy-8-alcoxy-octanoïque de formule VI avec la thiourée en présence d'acide bromhydrique concentré, ce qui donne l'acide R/S-gamma-liponique qu'on isole, ou bien
au stade de réaction D
on convertit l'acide R/S-gamma-liponique brut en l'acide R/S-alpha-liponique par oxydation par l'air en présence de quantités catalytiques de chlorure de fer-III et
au stade de réaction E
on soumet l'acide R/S-alpha-liponique à distillation continue dans un évaporateur à pellicule sous une pression de 0,05 à 0,1 mbar et à des températures de 130 à 160°C, et
au stade de réaction F
on purifié par cristallisation dans l'éther diisopropylique ou dans un mélange d'hexane et d'acétate d'éthyle l'acide R/S-alpha-liponique qui a déjà été soumis à purification préalable par distillation.

8. Acide 8-alcoxy-6-formyloxy-octanoïque de formule générale VII 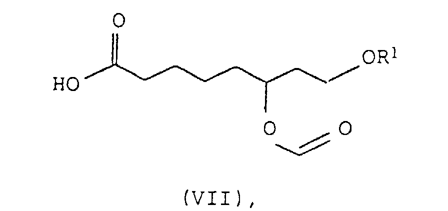 dans laquelle R¹ représente un groupe alkyle en C1-C3.

9. Procédé de préparation des acides 8-alcoxy-6-formyloxyoctanoïques de formule générale VII 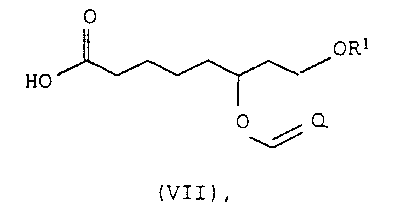 dans laquelle R¹ représente un groupe alkyle en C1-C3,
caractérisé par le fait que l'on fait réagir une 2-alcoxyéthyl-cyclohexanone de formule générale IV avec le peroxyde d'hydrogène dans l'acide formique.
